Europäisches Patentamt

European Patent Office

Office européen des brevets

Veröffentlichungsnummer: **0 296 339**

**A1**

# EUROPÄISCHE PATENTANMELDUNG

Anmeldenummer: **88107036.1**

Int. Cl.⁴ **A61B 6/03**

Anmeldetag: **02.05.88**

Die Bezeichnung der Erfindung wurde geändert (Richtlinien für die Prüfung im EPA, A-III, 7.3).

Priorität: **15.05.87 DE 8707038 U**

Veröffentlichungstag der Anmeldung:
**28.12.88 Patentblatt 88/52**

Benannte Vertragsstaaten:
**DE FR NL**

Anmelder: **Siemens Aktiengesellschaft Berlin und München**
**Wittelsbacherplatz 2**
**D-8000 München 2(DE)**

Erfinder: **Harke, Wolf-Udo, Dr.**
**Albert-Schweizer-Strasse 2**
**D-6902 Sandhausen(DE)**

**Kühlvorrichtung für einen Computertomographen.**

Zur Wärmeabfuhr der dem Strahlendetektor (3) zugeordneten Elektronik-Schaltkreise (11) sind deren Leiterplatten (18) auf ihrer detektornahen und detektorfernen Seite von je einer Kühlschlange (19, 20) eines geschlossenen Kühlkreislaufes bedeckt. Durch die Zwischenräume der Leiterplatten (18) wird Luft geblasen, welche die Wärme nach den Kühlschlangen (19, 20) abführt.

EP 0 296 339 A1

FIG 3

## Computertomograph

Die Erfindung betrifft einen Computertomograph mit einem Drehrahmen, auf dem ein Röntgenstrahler, ein Strahlendetektor und eine diesem nachgeschaltete Elektronik angeordnet sind.

Bei einem Computertomographen dieser Art besteht das Problem, die Wärme, die von den Komponenten auf dem Drehrahmen erzeugt wird, abzuführen. Hierzu ist es durch die deutsche Patentanmeldung 34 36 867 bekannt, zwischen dem Drehrahmen und dem feststehenden Teil einen Kanal vorzusehen, der von einem Kühlmittel durchströmt wird und in den Kühlrippen des Drehrahmens ragen.

Der Erfindung liegt die Aufgabe zugrunde, einen Computertomographen der eingangs genannten Art so auszubilden, daß eine effektive Kühlung der Elektronik sichergestellt ist.

Diese Aufgabe ist erfindungsgemäß dadurch gelöst, daß der Drehrahmen einen geschlossenen Kühlkreislauf trägt, bei dem der Verdampfer in Form von Kühlschlangen an der Elektronik und der Kondensator im Abstand vom Detektor mit der Elektronik angeordnet ist. Bei dem erfindungsgemäßen Computertomographen ist eine intensive Kühlung der Elektronik dadurch sichergestellt, daß ihr unmittelbar benachbart der Verdampfer eines Kühlkreislaufes angeordnet ist, über den die in der Elektronik entstehende Wärme abgeführt wird.

Eine besonders zweckmäßige Ausgestaltung der Erfindung mit schneller Wärmeabfuhr besteht bei dem Aufbau der Elektronik aus einer Reihe von Leiterplatten, die etwa auf den Fokus des Röntgenstrahlers ausgerichtet sind, darin, daß diese Reihe auf ihrer fokusnahen und fokusfernen Seite von je einer Kühlschlange abdeckt ist, wobei Kühlluft durch die Zwischenräume zwischen den Leiterplatten und die Kühlschlangen strömen kann.

Die Materialwahl für die Gehäuseteile der Elektronik wird ebenfalls im Hinblick auf eine optimale Wärmeabfuhr durchgeführt. Dabei können Gehäuseteile an ihrer Oberfläche auch schwarz ausgeführt sein.

Die Erfindung ist nachfolgend anhand eines in der Zeichnung dargestellten Ausführungsbeispieles näher erläutert. Es zeigen:

Fig. 1 eine schematische Darstellung eines Computertomographen nach der Erfindung, und

Fig. 2 bis 4 verschiedene Ansichten der Elektronik des Computertomographen gemäß Fig. 1 mit dem zugeordneten Verdampfer.

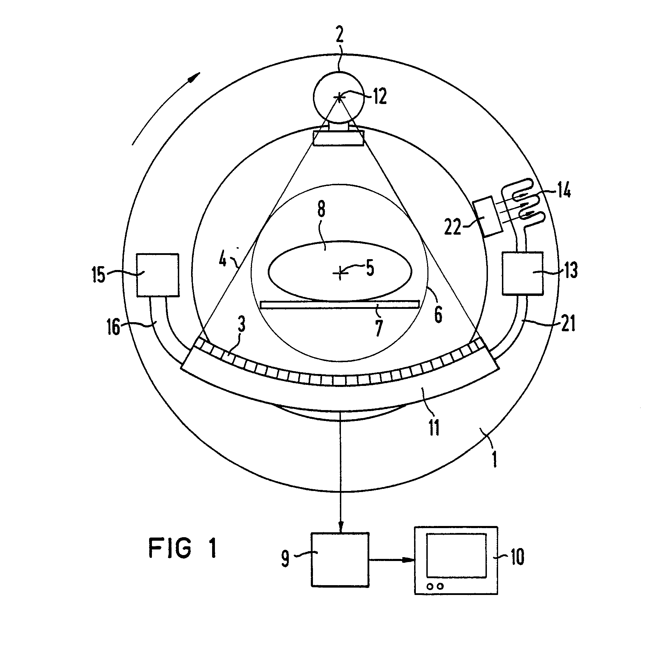

Der Computertomograph gemäß Fig. 1 weist einen Drehrahmen 1 auf, an dem ein Röntgenstrahler 2 und ein aus einer Reihe von Einzeldetektoren, z.B. aus 512 Einzeldetektoren, bestehender Strahlendetektor 3 befestigt sind. Dem Röntgenstrahler 2 ist eine Blende zur Einblendung eines fächerförmigen Röntgenstrahlenbündels 4 zugeordnet, dessen Randstrahlen bei der Drehung des Drehrahmens 1 um eine Achse 5 ein Meßfeld 6 tangieren. Im Meßfeld 6 liegt eine Patientenliege 7 mit dem zu untersuchenden Patienten 8.

Bei der Durchstrahlung des Patienten 8 unter verschiedenen Richtungen durch Drehung des Drehrahmens 1 werden von den Einzeldetektoren des Strahlendetektors 3 elektrische Signale erzeugt, aus denen ein Computer 9 ein Bild der untersuchten Transversalschicht des Patienten 8 berechnet und auf einem Sichtgerät 10 wiedergibt.

Dem Strahlendetektor 3 ist eine Elektronik 11 zugeordnet, die in einem Gehäuse angeordnet ist. Die Elektronik 11 enthält eine Reihe von Leiterplatten, die etwa auf den Fokus 12 des Röntgenstrahlers 2 ausgerichtet sind. Die Elektronik 11 dient insbesondere zur Integration der Ausgangssignale der Einzeldetektoren.

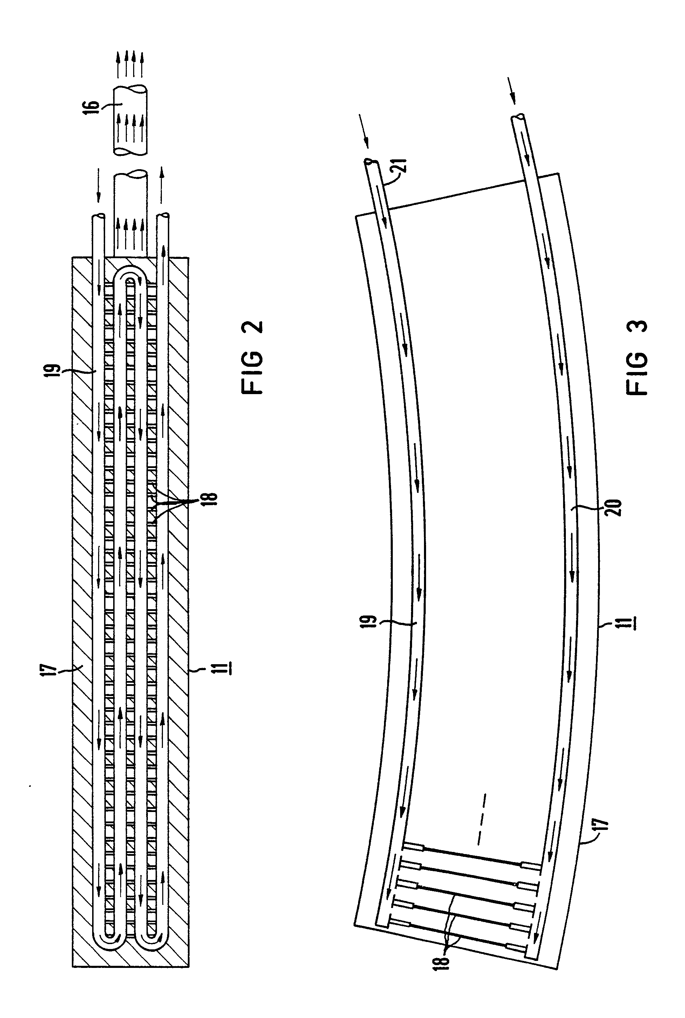

Zur Kühlung der Elektronik 11 ist ein geschlossener Kühlkreislauf mit einem Kompressor 13, einem Kondensator 14 und einem in Verbindung mit den Fig. 2 bis 4 noch näher beschriebenen Verdampfer in der Elektronik 11 auf dem Drehrahmen 1 vorgesehen. Ein Gebläse 15 bläst über eine Leitung 16 Luft durch die Zwischenräume zwischen den Leiterplatten der Elektronik 11 und sorgt für eine schnelle Wärmeabfuhr.

Die Fig. 2 zeigt das Gehäuse 17 der Elektronik 11 mit den Leiterplatten 18 in einer Ansicht von oben, d.h. vom Strahlendetektor 3 her. Eine mehrfach gewundene Kühlschlange 19 des Verdampfers des Kühlkreislaufes deckt die Leiterplatten 18 ab, zwischen denen Luft vom Gebläse 15 über die Leitung 16 hindurchgeblasen wird. Diese Luft wird durch die als Doppelleitung ausgeführte Leitung 16 auch abgeführt.

Die Fig. 3 zeigt eine Seitenansicht der Elektronik 11 bei geöffneten Gehäuse 17. Aus der Fig. 3 geht hervor, daß außer der Kühlschlange 19 noch eine weitere Kühlschlange 20 auf der dem Strahlendetektor 3 abgewandten Unterseite der Elektronik 11 angeordnet ist, die zusammen mit der Kühlschlange 19 den Verdampfer des Kühlkreislaufes bildet, der über eine Leitung 21 mit dem Kompressor 13 (Fig. 1) verbunden ist.

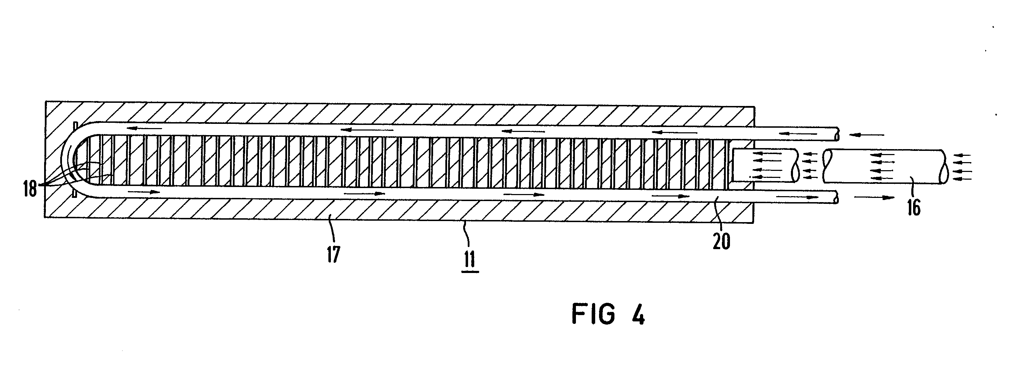

Die Fig. 4 zeigt die Ansicht der Elektronik 11 mit den Leiterplatten 18 und der Kühlschlange 20 von unten. In der Fig. 4 ist die Leitung 16 für die Zuführung der Luft des Gebläses 15 dargestellt. Die Leitung 16 ist eine Doppelleitung, durch die einerseits die Zuluft und andererseits die Abluft zurück zum Gebläse 15 geführt wird.

Das Gehäuse 17 kann aus Stahlblech besteht, wobei jedoch die Kühlschlangen 19, 20 wegen der besseren Wärmeleitfähigkeit auf einem Aluminiumblech montiert sein können. Dieses Aluminiumblech kann zur Erzielung eines geringen Wärmewiderstandes schwarz eloxiert sein. Der Boden und der Deckel des Gehäuses 11 können ebenfalls aus poliertem Stahlblech bestehen. Dadurch ist ein höherer Wärmewiderstand gewährleistet, so daß Wärme nach außen nicht abgestrahlt, sondern nur über den Kühlkreislauf entfernt wird. Außerdem ist so ein hochfrequenzdichtes Gehäuse gebildet.

Aus der Fig. 1 geht hervor, daß auch der Kondensator 14 durch ein auf dem Drehrahmen 1 angeordnetes Gebläse (22) gekühlt ist.

**Ansprüche**

1. Computertomograph mit einem Drehrahmen (1), auf dem ein Röntgenstrahler (2), ein Strahlendetektor (3) und eine diesem nachgeschaltete Elektronik (11) angeordnet sind, **dadurch gekennzeichnet,** daß der Drehrahmen (1) einen geschlossenen Kühlkreislauf (13, 14, 19, 20) trägt, bei dem der Verdampfer in Form von Kühlschlangen (19, 20) an der Elektronik (11) und der Kondensator (14) im Abstand vom Strahlendetektor (3) mit der Elektronik (11) angeordnet ist.

2. Computertomograph nach Anspruch 1, **dadurch gekennzeichnet,** daß die Elektronik (11) eine Reihe von Leiterplatten (18) aufweist, die etwa auf den Fokus (12) des Röntgenstrahlers (2) ausgerichtet sind und daß diese Reihe auf ihrer detektornahen und detektorfernen Seite von je einer Kühlschlange (19, 20) abgedeckt ist, wobei Kühlluft durch die Zwischenräume zwischen den Leiterplatten (18) und die Kühlschlangen (19, 20) strömen kann.

FIG 1

FIG 2

FIG 3

**FIG 4**

EP 0 296 339 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 026 812 (EMI LTD) <br> * Zusammenfassung; Seite 1, Zeilen 81-90,106-120; Seite 2, Zeilen 79-92; Figur 2 * <br> --- | 1 | A 61 B 6/03 |
| A | EP-A-0 109 206 (PICKER INT. INC.) <br> * Zusammenfassung; Seite 12, Zeile 30 - Seite 13, Zeile 5; Seite 14, Zeilen 1-5,16-23; Seite 15, Zeilen 3-25; Seite 17, Zeilen 6-15; Seite 18, Zeilen 4-17; Figuren 2-10 * <br> --- | 1,2 | |
| A | PATENT ABSTRACTS OF JAPAN, Band 6, Nr. 125 (P-127)[1003], 10. Juli 1982, Seite 100 P 127; & JP-A-57 50 673 (TOKYO SHIBAURA DENKI K.K.) 25-03-1982 * Zusammenfassung * <br> --- | 2 | |
| P,A | EP-A-0 225 964 (SIEMENS AG) <br> * Zusammenfassung; Spalte 2, Zeilen 1-45; Figuren 1-4 * <br> ----- | 1 | |
| | | | **RECHERCHIERTE SACHGEBIETE (Int. Cl.4)** <br><br> A 61 B |

Der vorliegende Recherchenbericht wurde für alle Patentansprüche erstellt

| Recherchenort | Abschlußdatum der Recherche | Prüfer |
|---|---|---|
| DEN HAAG | 08-08-1988 | RIEB K.D. |